# EUROPEAN PATENT SPECIFICATION

(11) **EP 1 932 550 B1**
(45) Date of publication and mention of the grant of the patent: **08.06.2016**
(21) Application number: 06797910.4
(22) Date of filing: 13.09.2006
(51) Int. Cl.: A61L 27/44, A61L 27/56, A61L 27/58

(54) **COMPOSITE POROUS MATERIAL**
ZUSAMMENGESETZTES PORÖSES MATERIAL
MATÉRIAU POREUX COMPOSITE

(30) Priority: 13.09.2005 JP 2005265340
(43) Date of publication of application: 18.06.2008
(73) Proprietor: TAKIRON CO., LTD., Osaka-shi Osaka (JP)
(72) Inventor: SHIKINAMI, Yasuo, Osaka-shi, Osaka (JP)
(74) Representative: Hoffmann Eitle
(86) International application number: PCT/JP2006/318160
(87) International publication number: WO 2007/032390

(56) References cited:
- EP-A1- 0 277 678
- EP-A1- 1 457 214
- WO-A1-03/092760
- WO-A1-03/092760
- WO-A2-2004/112855
- JP-A- 2005 067 966
- US-A1- 2004 258 732
- US-A1- 2005 169 893
- DEVIN J E ET AL: "THREE-DIMENSIONAL DEGRADABLE POROUS POLYMER-CERAMIC MATRICES FOR USE IN BONE REPAIR" JOURNAL OF BIOMATERIALS SCIENCE. POLYMER EDITION, VSP, UTRECHT, NL, vol. 7, no. 8, 1 January 1996 (1996-01-01) , pages 661-669, XP001057150 ISSN: 0920-5063

## Description

### TECHNICAL FIELD

The present invention relates to a composite porous object suitable for use in applications such as scaffolds for in vivo bone tissue regeneration, prosthetic materials, and bone fillers.

### BACKGROUND ART

The present applicant has already proposed a composite porous object as a material suitable for use in applications such as scaffolds for in vivo bone tissue regeneration. This composite porous object comprises a biodegradable and bioabsorbable polymer and bioceramic particles dispersed therein and has interconnected voids formed therein having a void diameter of 100-400 µm, part of the bioceramic particles being exposed on the surface and on the internal surface of the voids (patent document 1).

This composite porous object, when implanted in a defective part of a bone in a living body, progressively undergoes hydrolysis from the surface thereof as a result of contact with a body fluid of the living body and further progressively undergoes hydrolysis also from inner parts of the porous object due to the body fluid infiltrating into the inner parts through the interconnected voids. Simultaneously with this hydrolysis, the bioceramic particles, part of which are exposed on the surface of the composite porous object and on the internal surface of voids thereof, function to propagate a bone. As a result, bone tissues are propagated on the surface of the composite porous obj ect and the internal surface of voids thereof and grow toward inner parts in a relatively short time period. Namely, the composite porous object is an excellent material which is mostly replaced by bone tissues to regenerate the defective part of the living-body bone.
Patent Document 1: JP-A-2003-159321

Devin, J. E. et al, Journal of Biomaterials Science, vol. 7 no. 8, pp 661-669 discloses three-dimensional degradable porous polymer-ceramic matrices composed of polymeric microspheres for use in bone repair. The polymer matrix contained pores that are interconnected during polymer degradation. Mean macropore diameter ranged between 110 -150 µm, and mean micropore diameter ranged between 18-20 µm.

WO 03/092760 A1 discloses a structured composite as a carrier for tissue engineering and implant material of bones consisting of a mass of porous calcium phosphate granulates having a grain size of between 0.5 and 10 mm with macropores and micropores, and the macropores have an average diameter of between 50 and 500 µm and the micropores an average diameter of 0.5 to 5 µm.

US 2005/169893 A1 discloses a biocompatible bone graft material having a resorbable polymer and a resorbable inorganic material exhibiting macro, meso, and microporosities.

### DISCLOSURE OF THE INVENTION

### PROBLEM THAT THE INVENTION IS TO SOLVE

An object of the invention is to provide a composite porous object which not only enables bone tissues to propagate and grow on the internal surface of the voids but enables bone tissues to propagate and grow also in biodegradable and bioabsorbable polymer walls surrounding the voids to thereby attain a higher degree of replacement by bone tissues than that composite porous object, and which can be almost wholly replaced by bone tissues in a relatively short time period and can regenerate even a large defective part of a bone almost without fail.

### MEANS FOR SOLVING THE PROBLEM

In order to accomplish the object, the invention provides a composite porous object which comprises a biodegradable and bioabsorbable polymer containing bioactive bioceramic particles dispersed therein, characterized by having in inner parts thereof large voids having a void diameter of 40-600 µm and small voids having a void diameter of 1 µm or smaller, at least part of the large voids and at least part of the small voids constituting interconnected voids.

In such composite porous object, the biodegradable and bioabsorbable polymer walls surrounding the large voids have the small voids formed therein and in the surface thereof and the small voids have an average void diameter of 0.2 µm. It is preferred that 40% or more of all voids should constitute interconnected voids.

When the composite porous object of the invention is implanted, for example, as a scaffold for in vivo bone tissue regeneration in a defective part of a bone in a living body, a body fluid comes into contact with the surface of the composite porous object to hydrolyze the composite porous object from its surface. Simultaneously therewith, the body fluid infiltrates into inner parts of the composite porous object through interconnected large voids. The body fluid further infiltrates also into interconnected small voids present in the biodegradable and bioabsorbable polymer walls surrounding the large voids, through the biodegradable and bioabsorbable polymer walls surrounding the large voids. The body fluid thus hydrolyzes the biodegradable and bioabsorbable polymer walls from the internal surface of the large voids and from the internal surface of the small voids. Simultaneously with this hydrolysis, the osteoplastic cells such as osteoblasts contained in the body fluid proliferate and grow on the surface of the composite porous object and on the internal surface of the large voids and small voids due to the bone conductivity and bone-inducing ability of the bioactive bioceramic particles.

In particular, the small voids are effective in fixing and proliferating/differentiating the osteoplastic cells, e.g., osteoblasts, and bone growth factors which have infiltrated inside. Because of this, the composite porous object is progressively replaced by bone tissues conducting inward from the surf ace of the porous object. In addition, the biodegradable and bioabsorbable polymer walls surrounding the large voids present in inner parts of the composite porous object simultaneously undergo replacement by bone tissues conducting from the wall surface (i.e., from the internal surface of the large voids) into the walls and replacement by bone tissues conducting from the internal surface of the small voids in the walls toward surrounding parts. At the time when the composite porous object is wholly decomposed and assimilated to disappear, the composite porous object has been almost completely replaced by bone tissues. The composite porous object of the invention thus attains a high degree of replacement by bone tissues. Because of this, even when it is implanted in a large defective part of a bone of a living body, the composite porous object is almost completely replaced by bone tissues and can regenerate the defective part almost without fail.

In contrast, in the case where the biodegradable and bioabsorbable polymer walls surrounding large voids do not have small voids therein, the only replacement which the biodegradable and bioabsorbable polymer walls undergo is replacement by bone tissues conducting from the surface of the walls into the walls. Because of this, replacement by bone tissues does not sufficiently proceed before the disappearance of the walls. Consequently, a part having insufficient strength remains without being replaced by bone tissues. There is hence a possibility that the regeneration of a defective part of a living-body bone might be insufficient.

Especially when the defective part is large, this possibility is high.

On the other hand, in case where less than 40% of all voids are present as interconnected voids, there is a possibility that the proportion of large voids and small voids into which a body fluid infiltrates might be too low. There are hence cases where the biodegradable and bioabsorbable polymer walls surrounding the large voids in this composite porous object may be insufficiently hydrolyzed and insufficiently replaced by bone tissues. The composite porous object in which 40% or more of all voids constitute interconnected voids as in claim 2 is preferred because this porous object is free from such a fear.

### BRIEF DESCRIPTION OF THE DRAWINGS

[Fig. 1] Fig. 1 is an enlarged cut-edge view diagrammatically illustrating the structure of a cut edge of a composite porous object according to the invention.
[Fig. 2] Fig. 2 is a graph showing the distribution of void sizes (void diameters) of a composite porous object according to the invention.

### DESCRIPTION OF REFERENCE NUMERALS

1 large void
2 small void
3 biodegradable and bioabsorbable polymer wall surrounding large void

### BEST MODE FOR CARRYING OUT THE INVENTION

Embodiments of the invention will be described below in detail by reference to the drawings.

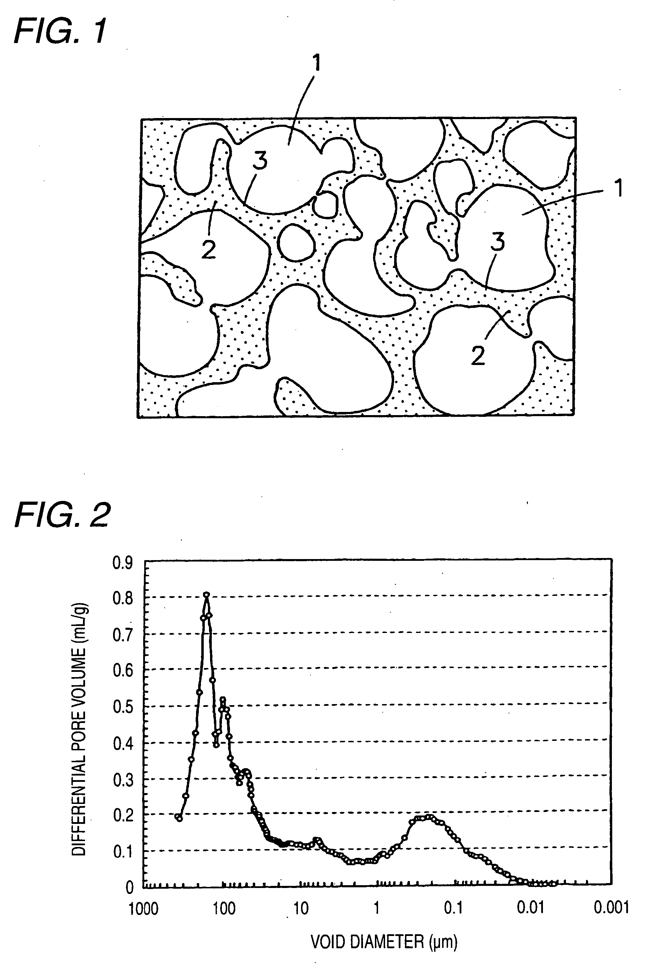

Fig. 1 is an enlarged cut-edge view diagrammatically illustrating the structure of a cut edge of a composite porous object according to the invention.

This composite porous object comprises a biodegradable and bioabsorbable polymer containing bioactive bioceramic particles dispersed therein. It has in inner parts thereof large voids 1 having a void diameter of 40-600 µm and small voids 2 having a void diameter of 1 µm or smaller. These small voids 2 are formed in the biodegradable and bioabsorbable polymer walls 3 surrounding the large voids 1 (in other words, in the framework matrix of the composite porous object) and in the surface of the walls. At least part of the large voids 1 and at least part of the small voids 2 constitute interconnected voids, and 40% or more of all voids constitute interconnected voids.

In Fig. 1, small voids appearing in the internal surface of large voids 1 (in the wall surface) are omitted in order to clearly show the large voids 1. However, small voids are formed also in the internal surface of the large voids 1 (in the wall surface).

The porosity of this composite porous object, i.e., the proportion by volume of all voids to the composite porous object, should be in the range of 20-90%. The reasons for this are as follows. In case where the porosity thereof is lower than 20%, the proportion of the biodegradable and bioabsorbable polymer constituting the composite porous object is so high that hydrolysis by a body fluid and replacement with bone tissues by propagation necessitate much time, resulting in a disadvantage that the regeneration of a defective part of a living-body bone is slow. In case where the porosity thereof exceeds 90%, the proportion of the biodegradable and bioabsorbable polymer is too low and this results in a disadvantage that the strength of the composite porous object itself and the strength of the regenerated bone part after replacement by bone tissues are insufficient. A preferred porosity is 50-85%, and a more preferred porosity is 60-80%. As long as the porosity thereof is within this range, hydrolysis and replacement by bone tissues proceed without delay and the strength is sufficient. Consequently, a defective part of a bone can be regenerated in a relatively short time period almost without fail.

The proportion of interconnected voids is preferably 40% or higher based on all voids as stated above. When the proportion of interconnected voids is 40% or higher, the large voids 1 and small voids 2 which permit body fluid infiltration thereinto are present in a sufficiently large amount. Because of this, the biodegradable and bioabsorbable polymer walls 3 surrounding the large voids in the composite porous object sufficiently undergo hydrolysis and replacement by bone tissues, whereby the object of the invention can be accomplished. A more preferred proportion of the interconnected voids is 60% or higher, and an even more preferred proportion thereof is 70% or higher. The upper limit of the proportion of the interconnected voids is up to the maximum value at which the formation of interconnected voids is technically possible. It may be 100% if possible.

The large voids 1 are ones having a void diameter of 40-600 µm. However, when suitability for the infiltration of a body fluid containing osteoplastic cells, e. g. , osteoblasts, is taken into account, the average diameter of the large voids 1 is preferably 100-300 µm. In case where the void diameter is smaller than 40 µm, the infiltration of a body fluid containing osteoplastic cells, e.g., osteoblasts, is slow. In contrast, when the void diameter is 40 µm or larger, especially 100 µm or larger, the infiltration of a body fluid containing osteoplastic cells, e.g., osteoblasts, is speedy. On the other hand, in case where the void diameter is larger than 600 µm, the rate of body fluid infiltration by a capillary phenomenon, etc. is low. In contrast, when the void diameter is 600 µm or smaller, especially 300 µm or smaller, the infiltration of a body fluid by a capillary phenomenon, etc. occurs speedily and the body fluid infiltrates into inner parts of the composite porous object through the large voids 1 in a short time period.

In contrast, the small voids 2 formed in the biodegradable and bioabsorbable polymer walls 3 surrounding the large voids 1 and in the surface of these walls are microvoids having a void diameter of 0.05-1 µm. The average void diameter thereof is around 0.2 µm. These small voids 2 have been formed evenly dispersedly in the biodegradable and bioabsorbable polymer walls 3. Some of the small voids are interconnected voids, while other small voids are closed voids.

The biodegradable and bioabsorbable polymer to be used as a material preferably is a polymer which has been put to practical use and ascertained to be safe and which is decomposed relatively rapidly, is not brittle even when in the form of a porous object, and is amorphous or a mixture of crystalline and amorphous states. For example, it preferably is poly(D,L-lactic acid), a block copolymer of L-lactic acid and D,L- lactic acid, a copolymer of a lactic acid and glycolic acid, a copolymer of a lactic acid and p-dioxanone, a copolymer of a lactic acid and caprolactone, a copolymer of a lactic acid and ethylene glycol, or a mixture of these . These polymers suitable for this use are ones having a viscosity-average molecular weight of 50,000-1,000,000 when the period of in vivo decomposition and assimilation is taken into account. Preferred of these are ones having a viscosity-average molecular weight of 100,000-300,000.

The bioceramic particles to be dispersed in the biodegradable and bioabsorbable polymer are not limited to sintered ones, and may be uncalcined and unsintered ones. Preferred bioceramic particles to be used are ones which are bioactive, have a satisfactory bone conductivity (sometimes with a bone-inducing ability) and a satisfactory affinity for the living body, and have the property of beingwholly assimilated by the living body. Examples thereof include particles of uncalcined and unsintered hydroxyapatite, uncalcined and unsintered calcium phosphate, dicalcium phosphate hydrate, β-tricalcium phosphate, tetracalcium phosphate, octacalcium phosphate, calcite, Ceravital, diopside, and natural coral. Also usable are ones obtained by adhering an alkaline inorganic compound, basic organic substance, or the like to the surface of those particulate materials. Exceedingly preferred of these are uncalcined and unsintered hydroxyapatite, β-tricalcium phosphate, and octacalcium phosphate. This is because they have high bioactivity, excellent bone conductivity, and high affinity for the living body, are low invasive, and are assimilated by the living body in a short time period.

As the particle diameter of the bioceramic particles increases, the activity thereof decreases and the complete assimilation thereof necessitates a longer period. It is therefore preferred to use ones having a particle diameter smaller than 10 µm, inparticular, ones having an average particle diameter of 3-5 µm.

It is preferred that the content of the bioceramic particles be regulated to 40-90% by mass. In case where the content thereof is lower than 40% by mass, the propagation of bone tissues based on bone conductivity is insufficient, resulting in a disadvantage that the degree of replacement by bone tissues decreases. In case where the content thereof exceeds 90% by mass, this composite porous object has a disadvantage that it is brittle and reduced in compressive and other mechanical strengths.

At least part of the bioceramic particles are frequently exposed on the surface of the walls 3 (internal surface of the large voids 1) and on the internal surface of the small voids 2. Such exposed state enables the propagation of bone tissues based on the bone conductivity of the bioceramic particles to begin in an early stage. This porous composite object is hence a more preferred porous composite object for use as a scaffold for in vivo bone tissue regeneration.

The composite porous object described above is produced, for example, by the following process. First, a biodegradable and bioabsorbable polymer is dissolved in a volatile solvent and bioceramic particles are mixed therewith to prepare a suspension. This suspension is formed into fibers by spraying or other technique to form a fibrous mass composed of entangled fibers. This fibrous mass is pressed into a fibrous mass molding having a desired shape. This fibrous mass molding is immersed in a volatile solvent such as methanol, ethanol, isopropanol, dichloroethane (dichloromethane), or chloroform. Upon this immersion, the fibrous mass molding comes into a semi-dissolved state and the fibers are substantially deprived of the fiber form while being fused to one another. The fibers thus become a matrix and the molding changes into a composite porous object having rounded voids derived from spaces among the fibers. The atmosphere is then gradually made vacuum to suck and remove the solvent present in this molding, whereby the target composite porous object is produced. It is preferred that this vacuum suction of the solvent be conducted by gradually bringing the atmosphere into a vacuum state over a time period of about from 10 seconds to 10 minutes.

A composite porous object produced by the process described above, specifically, the composite porous object obtained in Example 1, which will be given later, comprising poly(D,L-lactic acid) (viscosity-average molecular weight: 77,000) containing 70% by mass uncalcined and unsintered hydroxyapatite, was subjected to pore evaluation in accordance with JIS R1655 to examine the distribution of void sizes (void diameters). As a result, a distribution curve such as the graph shown in Fig. 2 was obtained. As apparent from this distribution curve, this composite porous object has not only a large amount of large voids having a void diameter of 40-300 µm but also a large amount of small voids of 1 µm or smaller. Such small voids of 1 µm or smaller are hardly present in ceramic porous objects obtained by sintering β-tricalcium phosphate. A composite porous object in which a differential pore volume peak for large voids is present at around 170 µm, the peak volume being 0.8 mL/g, and which has differential pore volumes for 40-300 µm large voids of 0.2 mL/g or larger, differential pore volumes for 50-250 µm large voids of 0.3 mL/g or larger, and differential pore volumes for 80-220 µm large voids of 0.4 mL/g or larger, as shown by the distribution curve, is preferred because it has many voids into which a body fluid is apt to infiltrate. Furthermore, a composite porous object in which a differential pore volume peak for small voids is present at around 0.2 µm, the peak volume being about 0.2 mL/g, and which has differential pore volumes for 0.05-1 µm small voids of 0.06 mL/g or larger, as shown by the distribution curve, is preferred because it has many small voids capable of fixing and proliferating/differentiating the osteoplastic cells and bone growth factors which have infiltrated thereinto.

Furthermore, the composite porous obj ect described above (average void diameter, 170 µm) comprising poly(D,L-lactic acid) containing 70% by mass uncalcined and unsintered hydroxyapatite was examined for the area of the internal surface of all voids by µCT. As a result, the internal-surface area was found to be 200 cm²/cm³, which is about two times the internal-surface area of a ceramic porous object obtained by sintering β-tricalcium phosphate (area of internal surface of all voids, 95 cm²/cm³). When the total surface area of the uncalcined and unsintered hydroxyapatite contained was calculated, it was found to be 1,780 cm²/cm³.

To have a large amount of small voids of 1 µm or smaller and have a large area of the internal surface of all voids as described above is exceedingly effective in accelerating bone induction and in fixing and proliferating/differentiating the osteoplastic cells, such as osteoblasts, and bone growth factors which have infiltrated into inner parts. In addition, as the degradable polymer progressively undergoes decomposition and assimilation by the action of the body fluid which has infiltrated into the small voids, particles of the uncalcined and unsintered hydroxyapatite are exposed and appear one after another, and the area in which osteoplastic cells and bone growth factors are induced increases more and more. Consequently, the growth of bone tissues is further accelerated. In this connection, it is said that the concentration of calcium ions and phosphate ions influences the osteoplastic cells. It is thought that particles of the uncalcined and unsintered hydroxyapatite contain calcium and phosphoric acid as components and, upon dissolution in a body fluid, give many micro islands the surface of which is surrounded by calcium ions and phosphate ions. The hydroxyapatite particles are thought to thus function more advantageously for the growth of bone tissues. Such an effect cannot be obtained with burned bioceramic particles which do not have in vivo assimilability.

As explained above, the composite porous object of the invention, when implanted as a scaffold for in vivo bone tissue regeneration in a defective part of a bone in a living body, functions by the following mechanism. As hydrolysis by a body fluid proceeds, the composite porous object is progressively replaced by bone tissues conducting inward from the surface of the composite porous object. Simultaneously with the replacement, the porous object further undergoes replacement by bone tissues conducting from the surface of the biodegradable and bioabsorbable polymer walls 3 surrounding the large voids 1 present in inner parts of the composite porous object (i.e., from the inner surface of the large voids 1) into the walls and replacement by bone tissues conducting from the internal surface of the small voids 2 in the walls 3 toward surrounding parts. The small voids are especially effective in fixing and proliferating/differentiating the osteoplastic cells, e.g., osteoblasts, and bone growth factors which have infiltrated inside. Consequently, a high degree of replacement by bone tissues is attained, and at the time when the composite porous object is wholly decomposed and assimilated to disappear, the composite porous object has been almost completely replaced by bone tissues. Therefore, this composite porous object is exceedingly useful as a scaffold for in vivo bone tissue regeneration, in particular, for the regeneration of a large defective bone part. Furthermore, the porous obj ect is suitable also for use in applications such as prosthetic materials, bone fillers, and cancellous bone substitutes.

Further specific Examples of the invention will be explained below.

### [EXAMPLE 1]

A polymer solution prepared by dissolving poly(D,L-lactic acid) (PDLLA) having a viscosity-average molecular weight of 77,000 (molar ratio of D-lactic acid/L-lactic acid, 50/50) in dichloromethane (concentration: 4 g of PDLLA/100 mL of dichloromethane) was mixed with a liquid mixture prepared by mixing particles of uncalcined and unsintered hydroxyapatite (u-HA) having an average particle diameter of 3 µm with ethanol. The resultant mixture was homogenized to thereby prepare a suspension in which 230 parts by weight of the u-HA was mixed with 100 parts by weight of the PDLLA.

Air brush HP-E (manufactured by Anest Iwata Corp.) was used as a sprayer. This sprayer was filled with the suspension, and this suspension was sprayed with 1.6 kg/cm² nitrogen gas on a polyethylene net (150 mesh) placed at a distance of about 120 cm therefrom to thereby form a fibrous mass. The fibrous mass was separated from the net. This fibrous mass had a fiber diameter of about 1.0 µm, fiber length of about 10-20 mm, and apparent specific gravity of 0.2.

This fibrous mass was cut into an appropriate size, packed into a cylindrical female mold having a diameter of 30 mm and a depth of 30 mm, and compressed with a male mold so that the fibrous mass came to have an apparent specific gravity of 0.5. Thus, a disk-form fibrous mass molding having a diameter of 30 mm and a thickness of 5 mm was obtained.

Subsequently, the fibrous mass molding was immersed in a solvent comprising dichloromethane mixed with ethanol to infiltrate the solvent into the molding. This molding was allowed to stand therein at 60°C for 10 minutes. Thereafter, the solvent present in inner parts of the molding was removed through suction by gradually bringing the atmosphere into a vacuum state. Thus, a composite porous object was obtained which had a diameter of 30 mm, thickness of 5 mm, and u-HA content of 70% by weight. This composite porous object was subjected to pore evaluation in accordance with JIS R1655 to examine the distribution of void sizes (void diameters). The results are as shown by the graph in Fig. 2 which was described above. This composite porous object had large voids having a void diameter of 40-600 µm and small voids having a void diameter of 1 µm or smaller.

### [EXAMPLE 2]

A disk-form fibrous mass molding having a diameter of 30 mm and a thickness of 5 mm was produced as a preform in the same manner as in Example 1. This preform was heated to 80°C in a Geer oven, subsequently placed in a chamber having a reduced-diameter part differing in diameter, and forced into a cylinder having a lower-part diameter of 10.6 mm.

Subsequently, this fibrous mass molding of a cylindrical rod shape was packed into a syringe having the same diameter and having through-holes in the peripheral wall. The molding was immersed for 10 minutes in a solvent (60°C) comprising dichloromethane containing 15% by weight methanol, while applying a pressure to the syringe from the upper and lower sides and thereby pressing it to such a degree that the height of the fibrous mass molding of a cylindrical rod shape did not change. Thereafter, the atmosphere was gradually brought into a vacuum state to remove the solvent by suction. Thus, a porous object was obtained.

### INDUSTRIAL APPLICABILITY

The composite porous object of the invention attains a high degree of replacement by bone tissues, and at the time when the composite porous object is wholly decomposed and assimilated to disappear, the composite porous object has been almost completely replaced by bone tissues. Because of this, the composite porous object is exceedingly useful as a scaffold for in vivo bone tissue regeneration, in particular, for the regeneration of a large defective bone part. Furthermore, the porous object is suitable also for use in applications such as prosthetic materials, bone fillers, and cancellous bone substitutes.

## Claims

1. A composite porous object which comprises a biodegradable and bioabsorbable polymer containing bioactive bioceramic particles dispersed therein, **characterized by** having in inner parts thereof large voids having a void diameter of 40-600 µm and small voids having a void diameter of 1 µm or smaller, at least part of the large voids and at least part of the small voids constituting interconnected voids, wherein the biodegradable and bioabsorbable polymer walls which surround the large voids have the small voids formed therein and in the surface thereof,
wherein the small voids have an average void diameter of 0.2 µm as evaluated by pore evaluation in accordance with JIS R1655.

2. The composite porous object according to claim 1, wherein 40 % or more of all voids constitute interconnected voids.

## Patentansprüche

1. Poröses Kompositobjekt, das ein biologisch abbaubares und bioresorbierbares Polymer, enthaltend bioaktive, darin dispergierte Biokeramik-Teilchen, umfasst, **dadurch gekennzeichnet, dass** es in seinen Innenteilen große Poren mit einem Porendurchmesser von 40-600 µm und kleine Poren mit einem Porendurchmesser von 1 µm oder weniger aufweist, wobei zumindest ein Teil der großen Poren und zumindest ein Teil der kleinen Poren miteinander verbundene Poren bilden, wobei die biologisch abbaubaren und bioresorbierbaren Polymerwände, die die großen Poren umgeben, die kleinen Poren aufweisen, die hierin und in der Oberfläche hiervon gebildet sind,
wobei die kleinen Poren einen mittleren Porendurchmesser von 0.2 µm, wie bewertet durch Poren-Bewertung gemäß JIS R1655, aufweisen.

2. Poröses Kompositobjekt gemäß Anspruch 1, wobei 40% aller Poren oder mehr die miteinander verbundenen Poren bilden.

## Revendications

1. Objet poreux composite qui comprend un polymère biodégradable et bioabsorbable contenant des particules de biocéramique bioactives dispersées dans celui-ci, **caractérisé en ce qu'**il possède, dans des parties internes de celui-ci, des grands espaces vides ayant un diamètre de vide de 40-600 µm et des petits espaces vides ayant un diamètre de vide de 1 µm ou moins, au moins une partie des grands espaces vides et au moins une partie des petits espaces vides constituant des espaces vides interconnectés, où les parois du polymère biodégradable et bioabsorbable qui entourent les grands espaces vides comportent les petits espaces vides formés dans celles-ci et dans la surface de celles-ci,
où les petits espaces vides possèdent un diamètre de vide moyen de 0,2 µm, tel qu'évalué par une évaluation des pores selon JIS R1655.

2. Objet poreux composite selon la revendication 1, dans lequel 40 % de tous les espaces vides ou davantage constituent des espaces vides interconnectés.
